# FASCICULE DE BREVET EUROPEEN

(11) **EP 0 717 979 B1**
(45) Date de publication et mention de la délivrance du brevet: **07.05.2003**
(21) Numéro de dépôt: 95402619.1
(22) Date de dépôt: 21.11.1995
(51) Int. Cl.: A61K 7/035

(54) **Composition cosmétique sous forme de poudre compacte et procédé de préparation**
Kompaktpulver, kosmetische Zusammensetzung und Verfahren zur Herstellung
Pressed powder cosmetic compositions and process for the preparation

(30) Priorité: 24.11.1994 FR 9414110
(43) Date de publication de la demande: 26.06.1996
(73) Titulaire: L'OREAL, 75008 Paris (FR)
(72) Inventeur: Lebras, Véronique, F-75002 Paris (FR); Mellul, Myriam, F-94240 l'Haye les Roses (FR)
(74) Mandataire: Dodin, Catherine

(56) Documents cités:
- EP-A- 0 447 286
- EP-A- 0 447 287
- EP-A- 0 605 284
- GB-A- 1 328 641
- D. F. WILLIAMS: "Cosmetics and Toiletries Industry" XP002100079 * page 1652, dernier alinéa * * page 166, alinéa 1 *

## Description

La présente invention concerne une composition, notamment cosmétique, se présentant sous forme d'une poudre compacte comprenant une phase grasse et une phase pulvérulente, ladite composition pouvant être utilisée en tant que produit de maquillage.
L'invention concerne également le procédé de fabrication de ladite composition.

Certaines compositions cosmétiques, telles que fond de teint, fard à paupières ou fard à joues, se présentent sous forme de poudre compacte, généralement constituées d'un liant gras et d'une phase pulvérulente contenant des pigments et/ou des charges.
Certaines compositions cosmétiques comprennent en outre une faible quantité de charge dite incompactable, notamment des microsphères de faible densité, car une telle charge confère à la composition un toucher très doux et non gras.
Par charge incompactable, on entend une matière première qui, à partir d'un certain pourcentage, qui dépendra de la matière en question, ne peut être compactée au moyen d'une presse mécanique.
Les compositions comprenant ces faibles quantités de charges incompactables présentent toutefois certains défauts : le produit compacté obtenu ne présente pas une bonne intégrité au cours de son stockage, le produit compacté ne présente pas une solidité aux chocs suffisante et/ou le produit compacté ne présente pas une surface plane convenable.
Par exemple, lorsque le produit compacté comprend des microsphères creuses en matériau thermoplastique de densité inférieure à 0,1 g/cm³, on observe l'apparition d'amorces de fragmentation et de morcellement lorsque le pourcentage de microsphères est supérieur à 1% en poids environ ; cette dégradation est le résultat de phénomènes de relaxation.

Diverses solutions ont été proposées pour remédier aux inconvénients ci-dessus. Dans EP 486 639, il est proposé de mélanger une phase pulvérulente et un liant dans un solvant non aqueux, de répartir la pâte fluide obtenue dans des moules appropriés puis d'évaporer le solvant. La phase particulaire contient des microsphères comportant une ou plusieurs cavités ouvertes ou fermées de façon à éviter tout retrait. Ce procédé présente plusieurs inconvénients : d'une part, les solvants étant souvent toxiques, on préfère éviter de les manipuler; de plus, le produit fini ne doit pas contenir de traces de solvant; d'autre part, le fait d'employer des solvants non aqueux empêche l'utilisation d'actifs hydrosolubles, comme par exemple les humectants.
Dans EP 447 286, on introduit dans une presse mécanique un liant et une phase pulvérulente contenant 0,02-5% de microsphères creuses en matériau thermoplastique ayant une masse spécifique inférieure à 0,1 g/cm³ et des dimensions inférieures à 30 µm. Toutefois, pour des quantités de microsphères supérieures à 1%, il se pose des problèmes d'intégrité du produit compacté au cours du stockage et des problèmes d'homogénéité de la répartition du liant sur un tel volume de charges. Or, c'est pour des proportions supérieures à 1% que l'on obtient des qualités cosmétiques vraiment originales. De plus, avec des microsphères de dimensions supérieures à 30 µm, on observe un phénomène de relaxation qui provoque des fragmentations dans le produit compacté.
Dans JP-A 62-53914, on décrit un procédé consistant à préparer un mélange aqueux de poudres cosmétiques et d'un composé macromoléculaire hydrosoluble en tant que liant, à couler le mélange dans un moule et à sécher par lyophilisation. On obtient ainsi un produit moulé de forme arbitraire, mais dont les qualités cosmétiques ne sont pas adéquates.

La présente invention a pour but de pallier les inconvénients de l'art antérieur et de proposer une composition se présentant sous forme d'une poudre compacte stable au stockage et ayant une bonne cohésion tout en contenant une quantité qui peut être importante de charges incompactables.
La composition obtenue est également solide, c'est-à-dire résistante aux chocs, et présente une surface plane et lisse.

La présente invention a pour objet une composition se présentant sous forme d'une poudre compacte comprenant une phase grasse et une phase pulvérulente, ladite phase pulvérulente comprenant un premier type de charge incompactable et au moins un second type de charge, lesdits premier et second types de charges étant différents, et étant choisis parmi le type lamellaire minéral, le type lamellaire organique, le type sphérique minéral et le type sphérique organique.

La présente invention a également pour objet un procédé de préparation de ladite composition dans lequel on disperse la phase pulvérulente dans une émulsion de type huile-dans-eau, on coule la dispersion obtenue dans un moule et on sèche par lyophilisation ladite dispersion.

Un avantage de l'invention est de permettre l'obtention d'une composition susceptible d'être présentée sous des formes diverses et variées, voire complexes, qu'il était difficile d'obtenir de façon reproductible selon l'état de la technique. En effet, selon une première technique de l'art antérieur, on moule à la presse des charges pulvérulentes en présence d'un liant mais il est alors difficile d'obtenir une pression constante en tous les points du moule si le moule a une forme compliquée ; par conséquent, la poudre compacte obtenue présente des zones de fragilité. Selon la seconde technique de l'art antérieur, on coule, dans un moule, un mélange sous forme d'une pâte fluide comportant un liant; lorsque la pâte est préparée par mélange avec de l'eau, des phénomènes de retrait apparaissent au cours du séchage et la poudre compacte obtenue est déformée et ne présente pas de surface plane. L'invention permet de pallier ces inconvénients et d'obtenir une composition de formes diverses.

Un autre avantage de l'invention est de permettre l'obtention d'une composition présentant une texture nouvelle, de toucher très doux et non gras. Un autre avantage de l'invention est de permettre l'obtention d'une composition présentant des qualités cosmétiques remarquables, grâce à la présence d'au moins deux charges de type différent.

Dans la suite de la présente description, les pourcentages sont donnés en poids sauf indication contraire.

La composition selon l'invention se présente donc sous la forme d'une poudre compacte comprenant une phase grasse et une phase pulvérulente.
La phase pulvérulente comprend au moins une première charge incompactable et au moins une seconde charge, qui peut être compactable ou incompactable, chaque charge étant choisie parmi les charges de type lamellaire minéral, les charges de type lamellaire organique, les charges de type sphérique minéral et les charges de type sphérique organique, lesdites première et seconde charges étant de type différent.

Chaque type de charges permet d'apporter des qualités particulières et différentes à la composition selon l'invention. Ainsi, par exemple, les charges de type lamellaire minéral apportent généralement de la douceur, les charges de type sphérique minéral apportent généralement un bon délitage et les charges sphérique organique ont généralement un rôle structurant et apportent de la douceur. Pour obtenir une composition possédant de bonnes propriétés cosmétiques, il est donc nécessaire de mélanger au moins deux charges de type différent.

Parmi les charges de type minéral lamellaire, on peut citer :
- les talcs ou silicates de magnésium hydratés, sous forme de particules de dimensions généralement inférieures à 40 µm;
- les micas ou aluminosilicates de compositions variées et qui se présentent sous forme d'écailles ayant des dimensions de 2 à 200 µm, de préférence 5-70 µm et une épaisseur de 0,1 à 5 µm, de préférence de 0,2-3 µm, ces micas pouvant être d'origine naturelle (par exemple muscovite, margarite, roscoelite, lipidolite, biotite) ou d'origine synthétique. Ils sont généralement transparents et permettent de conférer à la peau un aspect satiné;
- les argiles telles que les séricites, qui appartiennent à la même classe chimique et cristalline que la muscovite mais dont les propriétés organoleptiques sont proches du talc;
- le kaolin ou silicate d'aluminium hydraté, qui se présente sous la forme de particules de formes isotropes ayant des dimensions généralement inférieures à 30µm et qui possèdent de bonnes propriétés d'absorption des corps gras;
- les nitrures de bore.
Ces charges sont généralement compactables.

Toutefois, parmi ces charges de type lamellaire minéral, certaines sont incompactables. On peut ainsi citer
- certains talcs, tels que le 'Talc K1' de la société NIPPON ou le 'Talc Extra Steamic OOS' de la société LUZENAC;
- certaines séricites, telles que la 'Séricite BC282' de la société WHITTAKER;
- la plupart des micatitanes lorsqu'on les utilise à un fort pourcentage, parmi lesquels on peut citer le mica-nanotitane 'Coverleaf PC 2055M' de la société IKEDA.

Parmi les charges compactables de type lamellaire organique, on peut citer les poudres de polymères de tétrafluoroéthylène, telles que le 'Fluon' de la société MONTEFLUOS, ou le 'Hostaflonq' de la société HOECHST.

Parmi les charges incompactables de type lamellaire organique, on peut citer la lauroyl-lysine 'Aminope LL-11' de la société AJINOMOTO.

Parmi les charges compactables de type sphérique minéral, on peut citer :
- les oxydes de zinc et de titane, généralement utilisés sous la forme de particules ayant des dimensions ne dépassant pas quelques micromètres (ou même inférieures à 1 µm dans le cas de l'oxyde de titane), en particulier des dioxydes de titane sphérique comme le 'SPHERITITAN' de la société IKEDA; ces oxydes ont un toucher onctueux, un bon pouvoir couvrant et une opacité importante ;
- le carbonate de calcium précipité qui, sous forme de particules de dimensions supérieures à 10 µm, a un toucher onctueux et permet d'obtenir un aspect mat ;
- le carbonate et l'hydrocarbonate de magnésium, qui possèdent, notamment, des propriétés de fixation des parfums ;
- la silice sphérique non poreuse et
- l'hydroxyapatite.

Parmi les charges incompactables de type minéral sphérique, on peut citer :
- les microsphères de silice à porosité ouverte ou, de préférence, les microsphères de silice creuses, telles que les 'SILICA BEADS' de la société MAPRECOS, ces microsphères étant avantageusement imprégnées d'un actif cosmétique, et
- les microcapsules de verre ou de céramique 'MACROLITE' de la société 3M.

Parmi les charges compactables de type organique sphérique, on peut citer :
- les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; ces savons, présents généralement sous la forme de particules ayant des dimensions inférieures à 10 µm, ont un toucher onctueux et facilitent l'adhérence de la poudre à la peau;
- les poudres de polymères synthétiques non expansés, tels que le polyéthylène, les polyesters (par exemple isophtalate ou téréphtalate de polyéthylène) et les polyamides (par exemple le Nylon), sous la forme de particules ayant des dimensions inférieures à 50 µm, qui possèdent des propriétés absorbantes et permettent de conférer à la peau un aspect velouté ;
- les poudres de polymères synthétiques, réticulés ou non, sphéronisées, comme les poudres de polyamides telles que les poudres de poly-β-alanine ou de Nylon, par exemple la poudre 'Orgasol' de la société ATOCHEM, des poudres d'acide polyacrylique ou polyméthacrylique, des poudres de polystyrène réticulé par le divinylbenzène et des poudres de résine de silicone, et
- des poudres de matériaux organiques d'origine naturelle comme les amidons de maïs, de blé ou de riz.

Parmi les charges incompactables de type organique sphérique, on peut citer :
- les microsphères microporeuses de polymères, qui ont une structure analogue à celle d'une éponge; elles ont, en général, une surface spécifique d'au moins 0,5 m²/g et, en particulier, d'au moins 1 m²/g, ladite surface spécifique n'ayant pas de limite supérieure autre que celle résultant de la possibilité pratique de réaliser des microsphères de porosité très élevée : la surface spécifique peut, par exemple, atteindre 1 000 m²/g ou même davantage. On peut citer les microsphères de polymères acryliques, telles que celles en copolymère d'acrylate réticulé 'Polytrap' de la société DOW CORNING, et celles de polyméthacrylate de méthyle 'MICROPEARL M' ou 'MICROPEARL M 100' de la société SEPPIC; ces microsphères microporeuses peuvent avantageusement être imprégnées, notamment par des actifs cosmétiques : on peut citer, à cet égard, les microsphères de copolymères de styrène-divinylbenzène vendues sous la dénomination commerciale 'PLASTIC POWDER FPSQ' par la société TOSHIKI, qui sont imprégnées de squalane qui est un actif cosmétique émollient;
- les microcapsules de polymères; qui comportent une seule cavité fermée et forment un réservoir, qui peut contenir un liquide, notamment un actif cosmétique; elles sont préparées par des procédés connus tels que ceux décrits dans le brevet US-A 3 615 972 et EP-A 0 56219 Elles peuvent être réalisées, par exemple, en polymères ou copolymères d'acides, d'amines ou d'esters monomères à insaturation éthylénique, en polymères urée-formaldéhyde, en polymères ou copolymères de chlorure de vinylidène; à titre d'exemple, on peut citer les microcapsules faites de polymères ou copolymères d'acrylate ou de méthacrylate de méthyle, ou encore de copolymères de chlorure de vinylidène et d'acrylonitrile; parmi ces derniers, on indiquera, notamment, ceux qui contiennent, en poids 20-60% de motifs dérivés de chlorure de vinylidène, 20-60% en poids de motifs dérivés d'acrylonitrile et 0-40% en poids d'autres motifs tels que des motifs dérivés d'un monomère acrylique et/ou styrénique; on peut également utiliser des polymères ou copolymères acryliques réticulés, par exemple dans le cas de polymères comportant un groupement carboxylique, par des diols servant d'agents réticulants; à titre d'exemple, on peut citer les microcapsules en copolymère de chlorure de vinylidène-acrylonitrile 'EXPANCEL' de la société Kemanord Plast, les microcapsules 'Q-MAX' de la société Q-MAX et les microcapsules '3 M' de la société 3M.

La phase pulvérulente peut comprendre 1-100% de charges incompactables, de préférence 60-100%, et 0-99% de charge compactable, de préférence 0-40%, lesdits pourcentages étant donnés par rapport à la phase pulvérulente.

Lorsque les charges incompactables ont une densité très faible, notamment inférieure à 0,1 g.cm⁻³, elles sont de préférence présentes à raison de 2-10% par rapport à la composition finale.
Lorsque les charges incompactables ont une densité comprise entre 0,15 et 0,5 g.cm⁻³, elles sont de préférence présentes à raison de 2-40% par rapport à la composition finale.
Lorsque les charges incompactables ont une densité plus élevée, notamment supérieure à 0,5 g.cm⁻³, elles sont de préférence présentes à raison de 30-90% par rapport à la composition finale.

La phase pulvérulente peut contenir, outre les charges, des pigments, de préférence en une quantité de 0-50% par rapport au poids total de la composition finale. Ces pigments peuvent être choisis parmi les pigments minéraux, les pigments organiques et les pigments nacrés.
Parmi les pigments minéraux, on peut citer, par exemple le dioxyde de titane (rutile ou anatase), éventuellement traité en surface; les oxydes de fer noir, jaune, rouge et brun; le violet de manganèse; le bleu outremer; l'oxyde de chrome éventuellement hydraté ; le bleu ferrique.
Parmi les pigments organiques, on peut citer, par exemple, les pigments D & C red, D & C orange, D & C yellow, le noir de carbone, et les laques à base de carmin de cochenille.
Les pigments nacrés peuvent être choisis, notamment, parmi les pigments nacrés blancs tels que le mica recouvert d'oxyde de titane ou l'oxychlorure de bismuth; les pigments nacrés colorés tels que le micatitane avec des oxydes de fer, le micatitane avec du bleu ferrique ou de l'oxyde de chrome, le micatitane avec un pigment organique du type précité, ainsi que les pigments à base d'oxychlorure de bismuth.

La composition comprend également une phase grasse. Cette phase grasse peut comprendre des huiles et/ou des cires d'origine animale, végétale, minérale ou synthétique, seules ou en mélanges.

Parmi les huiles utilisables, on peut citer l'huile de vison, l'huile de tortue, l'huile de soja, l'huile de pépins de raison, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide; les huiles d'hydrocarbures, telles que les huiles de paraffine, le squalane, la vaseline; les esters gras, tels que le myristate d'isopropyle, le palmitate d'isopropyle, le stéarate de butyle, le laurate d'hexyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéa-rate de glycérine ou de diglycérine; les huiles de silicone telles que les polyméthylsiloxanes, les polyméthylphénylsiloxanes, les polysiloxanes modifiés par des acides gras, des alcools gras ou des polyoxyalkylènes, les silicones fluorées, les huiles perfluorées; les acides gras supérieurs tels que l'acide myristique, l'acide palmitique, l'acide stéarique, l'acide béhénique, l'acide oléique, l'acide linoléique, l'acide linolénique ou l'acide isostéarique; les alcools gras supérieurs tels que le cétanol, l'alcool stéarylique ou l'alcool oléique.

Parmi les cires utilisables, on peut citer les cires d'abeille, les cires de lanoline et les cires d'insectes de Chine; les cires de Carnauba, de Candelila, d'ouricurry, les cires de fibres de liège, les cires de canne à sucre, les cires du Japon, les cires de jojoba hydrogénées et les huiles hydrogénées telles que l'huile de tournesol hydrogénée, l'huile de ricin hydrogénée, l'huile de coprah hydrogénée et la lanoline hydrogénée; les paraffines, les cires microcristallines, les cires de Montan et les ozokérites; les cires de polyéthylène, les cires obtenues par la synthèse de Fisher-Tropsch, les copolymères cireux ainsi que leurs esters, et les cires de silicone telles que les polyalcoxy et polyalkylsiloxanes.

La phase grasse peut, en outre, comprendre des additifs tels que des actifs cosmétiques lipophiles et/ou des ingrédients liposolubles généralement utilisés en cosmétique comme les parfums. De préférence, ces additifs peuvent être présents en une quantité de 0-20% par rapport au poids total de la phase grasse.

La présente invention concerne également un procédé de préparation d'une composition telle que définie ci-dessus, dans lequel on disperse la phase pulvérulente dans une émulsion de type huile-dans-eau, on coule la dispersion obtenue dans un moule et on sèche par lyophilisation ladite dispersion. On peut ensuite éventuellement démouler la poudre compacte sèche obtenue.

Un avantage du procédé selon l'invention est qu'il permet la réalisation de poudres compactes comprenant un fort taux de charges, notamment incompactables, poudres qu'il n'était pas possible d'obtenir de manière satisfaisante par les procédés de l'état de la technique.
Un autre avantage du procédé selon l'invention est qu'il permet d'introduire des additifs hydrosolubles dans la composition finale, via la phase aqueuse de l'émulsion.
Ces additifs peuvent être, par exemple, des adjuvants de formulation et/ou des actifs cosmétiques.
Parmi les adjuvants de formulation, on peut citer les épaississants tels que les gommes naturelles, par exemple la gomme arabique, la gomme adragante, la gomme de guar; les dérivés de la cellulose, les pectines comme les dérivés de l'acide alginique et du carraghen, les bentonites et les silices colloïdales, les polysaccharides, des macromolécules synthétiques notamment à groupements vinyliques ou acryliques, des matières amylacées, des dérivés phosphorylés d'alcool aliphatique hydroxylé, des triglycérides naturels ou synthétiques estérifiés; les conservateurs tels que le méthylparaben; les agents de modification du pH tels que la triéthanolamine.
Parmi les actifs cosmétiques, on peut citer les agents antioxydants ou anti-radicaux libres; les agents hydratants ou humectants tels que la glycérine et le collagène; les agents filtres UV tels que la benzophénone.
Ces additifs hydrosolubles peuvent être présents dans la composition finale en une quantité de 0-20%, de préférence 0,5-10%.

On décrit ci-après un mode particulier et préféré de mise en oeuvre du procédé selon l'invention.
Selon ce mode préféré, on prépare une émulsion de type huile-dans-eau en présence d'un agent tensioactif, tel que le stéarate de triéthanolamine ou les mélanges d'acide stéarique et de triéthanolamine. La quantité d'agent tensioactif utilisée est de préférence de 5-15% par rapport au poids total de la dispersion, et d'au plus 30%.
On introduit ensuite progressivement dans l'émulsion obtenue, les différents constituants de la phase pulvérulente, de façon à obtenir une dispersion. La dispersion obtenue peut comprendre 0,1-30% de phase grasse, 0,1-65% de phase pulvérulente et 30-70% de phase aqueuse. De préférence, la dispersion comprend 1-15% de phase grasse, 1-45% de phase pulvérulente et 35-55% de phase aqueuse.

Avant moulage, la dispersion présente, de préférence, une viscosité de 0,5-30 Pa.s, mesurée à 25°C à l'aide d'un viscosimètre rotatif.
En effet, lorsque la viscosité est inférieure à 0,5 Pa.s, la composition obtenue après séchage a tendance à s'affaisser et à être molle. Lorsque la viscosité est supérieure à 30 Pa.s, on a quelques difficultés pour remplir les moules.

On introduit la dispersion dans un moule de forme très variée : parallélépipédique, cylindrique, sphérique, hémisphérique, tronconique ou toute forme esthétique désirée.
On introduit ensuite le moule dans un lyophilisateur. La congélation est, de préférence, effectuée à une vitesse très faible, de l'ordre de 0,5°C/min, jusqu'à une température inférieure à la température de fusion commençante de la dispersion qui est généralement comprise entre -15°C et -5°C, pour obtenir une cristallisation très fine de l'eau.
Lorsque le produit est entièrement congelé, il y a sublimation primaire des cristaux de glace; la pression de sublimation est déterminée par la température de fusion commençante de la dispersion, température que l'on ne doit de préférence pas dépasser pendant le cycle de lyophilisation afin qu'il n'y ait pas de fusion interstitielle. De préférence, on peut sublimer à une pression de 40 Pa (0,4 mbar) pour une température de fusion commençante d'environ -12°C.
La sublimation a lieu jusqu'à ce que le dernier cristal de glace soit sublimé. A ce moment-là, la température du produit s'élève car le phénomène endothermique de sublimation n'a plus lieu.
On passe alors au stade de dessiccation secondaire: à ce stade, la pression est maintenue très basse, à environ 1 Pa et l'on amène la température à environ 25°C pour évacuer les dernières traces d'eau.
On démoule ensuite éventuellement la composition sous forme de poudre compacte sèche obtenue.

D'une manière générale, la composition selon l'invention peut comprendre, après lyophilisation, 2-98% de phase pulvérulente, de préférence 60-97%, et 2-98% de phase grasse, de préférence 3-40%.
Dans une forme préférée de composition selon l'invention, ladite composition comprend 45-97% de charge incompactable, 0-40% de charge compactable et 3-30% de phase grasse.

La composition ainsi obtenue peut donc se présenter sous forme de coupelles, de sticks, de cylindres, ou sous toute autre forme complexe.
Elle présente l'aspect d'une poudre compactée usuelle bien qu'elle n'ait pas été obtenue par compactage à l'aide d'une presse mécanique.

La composition obtenue grâce au procédé selon la présente invention présente, notamment lorsqu'on l'observe en coupe au microscope, un réseau poreux tridimensionnel. On peut envisager, sans être tenu par cette explication, que ce réseau est dû à la présence d'une phase grasse et/ou d'une phase aqueuse gélifiée, dans la composition, avant sa lyophilisation.
Le procédé selon l'invention permet donc d'obtenir un réseau plus solide, ainsi qu'une meilleure répartition des matières pulvérulentes, d'où une composition finale plus confortable sur la peau, sans sensation de gras, bien qu'elle puisse contenir une quantité important de phase grasse.

De préférence, la composition selon l'invention, obtenue après lyophilisation, présente une porosité interne supérieure à 2 m²/g, voire supérieure à 3 m²/g, et préférentiellement de l'ordre de 4 à 100 m²/g.

La présente invention est illustrée plus en détail dans les exemples suivants.

### EXEMPLE 1

On prépare une poudre compacte de maquillage ayant la composition suivante :
*phase pulvérulente*
   - Microcapsules de silice (SILICA BEADS 150 de MAPRECOS)
      (charge sphérique minérale incompactable) 25%
   - Microcapsules en copolymère chlorure de vinylidène-acrylonitrile (EXPANCEL 551 DE de KEMANORD PLAST )
      (charge sphérique organique incompactable) 2%
   - Pigments (oxydes de fer jaune, rouge et noir, dioxyde de titane) 5%
*phase grasse*
   - Huile de parléam 5%
   - Stéarate de glycéryle 2,2%
   - Emulsionnant (acide stéarique et triéthanolamine) 3,3%
*phase aqueuse*
   - Eau 57,2%
   - Conservateur 0,3%

On prépare une émulsion huile-dans-eau de manière classique dans un mélangeur de type MORITZ en introduisant la phase grasse dans la phase aqueuse à 80°C. Une fois formée, on introduit progressivement sous agitation la phase pulvérulente, de façon à obtenir une dispersion ayant une viscosité de 6 Pa.s.
On dispose la dispersion obtenue dans des moules que l'on introduit dans un lyophilisateur et on abaisse la température jusqu'à -40°C avec une vitesse de congélation de 0,5° C/min. Lorsque le produit est entièrement congelé, on amène la pression à 40 Pa de façon à provoquer la sublimation primaire des cristaux de glace.
La composition obtenue après démoulage se présente sous forme sèche et non hygroscopique, de toucher agréable et ne présente ni déformation ni défaut de surface.
On peut aisément prélever la poudre pour application à l'aide d'un pinceau.

### EXEMPLE 2

On prépare comme dans l'exemple 1, une poudre compacte ayant la composition suivante :
*phase pulvérulente*
   - Microsphères de copolymères de styrène/divinylbenzène ayant absorbé 3% de squalane (PLASTIC POWDER de TOSHIKI)
      (charge sphérique organique incompactable) 10%
   - Microcapsules de silice (SILICA BEADS 150 de MAPRECOS)
      (charge sphérique minérale incompactable) 10%
   - Mica (charge minérale lamellaire compactable) 10%
   - Pigments 5%
*phase grasse*
   - Huile de parléam 5%
   - Stéarate de glycéryle 2,2%
   - Emulsionnant 3,3%
*phase aqueuse*
   - Eau 54,2%
   - Conservateur 0,3%

La composition est préparée comme dans l'exemple 1.
Après lyophilisation, on obtient une masse solide sèche ne présentant ni déformation, ni défaut de surface et non hygroscopique. Cette poudre compacte à un toucher doux et l'on peut facilement prélever la poudre avec un pinceau pour le maquillage.

### EXEMPLE 3 (contre-exemple)

A titre comparatif, on prépare une composition ne contenant qu'une charge incompactable, de type minérale sphérique. Cette composition comprend :
*phase pulvérulente*
   - Microcapsules de silice (SILICA BEADS 150 de MAPRECOS)
      (charge sphérique minérale incompactable) 25%
   - Pigments 5%
   *phase grasse*
   - Huile de parléam 5%
   - Stéarate de glycéryle 2,2%
   - Emulsionnant 3,3%
*phase aqueuse*
   - Eau 59,2%
   - Conservateur 0,3%

La composition est préparée comme dans l'exemple 1.
La poudre compacte obtenue ne présente pas un bon délitage ; le toucher est beaucoup plus gras et elle a tendance à cirer.

### EXEMPLE 4

On prépare une poudre compacte pour le visage ayant la composition suivante:
*phase pulvérulente*
   - Microcapsules de silice (SILICA BEADS 150 de MAPRECOS)
      (charge sphérique minérale incompactable) 25%
   - Microcapsules en copolymère chlorure de vinylidène-acrylonitrile (EXPANCEL 551 DE de KEMANORD PLAST)
      (charge sphérique organique incompactable) 2%
   - Pigments 5%
*phase grasse*
   - Huile de parléam 5%
   - Stéarate de glycéryle 2,2%
   - Emulsionnant 3,3%
*phase aqueuse*
   - Eau 52,2%
   - Glycérine 5%
   - Conservateur 0,3%

On prépare la composition de manière similaire à l'exemple 1.
La poudre obtenue se prélève facilement avec un pinceau pour le maquillage et n'est pas rêche sur la peau.

### EXEMPLE 5

On réalise la composition selon l'exemple 1 et la sèche par deux moyens différents :
. à température ambiante
. à l'étuve à 60°C.

On constate que les deux produits obtenus par l'un ou l'autre de ces moyens sont fissurés et présentent un toucher très gras.
Pour mémoire, dans l'exemple 1 dans lequel le séchage est effectué par lyophilisation, le produit obtenu est intact et de toucher non gras.

### EXEMPLE 6 (contre-exemple)

A titre comparatif, on prépare une composition ne contenant qu'une charge de type minérale lamellaire. Cette composition comprend :
*phase pulvérulente*
   - Talc 25%
   - Pigments 5%
*phase grasse*
   - Huile de parléam 5%
   - Stéarate de glycéryle 2,2%
   - Emulsionnant 3,3%
*phase aqueuse*
   - Eau 59,2%
   - Conservateur 0,3%

La composition est préparée comme dans l'exemple 1.

On constate que la composition obtenue présente une cristallisation en feuillets, c'est-à-dire une surface hétérogène. De plus, elle ne se délite pas facilement et a tendance à cirer.

### EXEMPLE 7

On prépare, selon l'exemple 1, une composition comprenant
*phase pulvérulente*
   - Microcapsules de silice (SILICA BEADS 150 de MAPRECOS)
      (charge sphérique minérale incompactable) 26%
   - Microcapsules en copolymère chlorure de vinylidène-acrylonitrile (EXPANCEL 551 DE de KEMANORD PLAST )
      (charge sphérique organique incompactable) 13,25%
   - Mica 2%
*phase grasse*
   - Huile de parléam 5%
   - Stéarate de glycéryle 2,2%
   - Emulsionnant (acide stéarique et triéthanolamine) 3,3%
*phase aqueuse*
   - Eau 47,95%
   - Conservateur 0,3%

La mesure de porosité interne, par BET, donne le résultat suivant : 5 m²/g.

## Revendications

1. Procédé de préparation d'une composition comprenant une phase grasse et une phase pulvérulente, ladite phase pulvérulente comprenant un premier type de charge, incompactable, et un second type de charge, lesdits premier et second types de charges étant différents, et étant choisis parmi le type lamellaire minéral, le type lamellaire organique, le type sphérique minéral et le type sphérique organique, procédé dans lequel on prépare une émulsion de type huile-dans-eau de la phase grasse dans une phase aqueuse, on disperse la phase pulvérulente dans ladite émulsion, on coule la dispersion obtenue dans un moule et on sèche par lyophilisation ladite dispersion.

2. Procédé selon la revendication 1, dans lequel la dispersion obtenue après lyophilisation est démoulée de manière à obtenir une poudre compacte.

3. Procédé selon l'une des revendications précédentes, dans lequel l'émulsion est préparée en présence d'agent tensioactif, de préférence en une quantité de 5-15% par rapport au poids total de la dispersion.

4. Procédé selon l'une des revendications précédentes, dans lequel la dispersion avant moulage comprend 0,1-30% de phase grasse, 0,1-65% de phase pulvérulente et 30-70% de phase aqueuse.

5. Procédé selon l'une des revendications précédentes, dans lequel la dispersion avant moulage comprend 1-15% de phase grasse, 1-45% de phase pulvérulente et 35-55% de phase aqueuse.

6. Procédé selon l'une des revendications précédentes, dans lequel la dispersion présente, avant moulage, une viscosité de 0,5-30 Pa.s, mesurée à 25°C à l'aide d'un viscosimètre rotatif.

7. Procédé selon l'une des revendications précédentes, dans lequel la lyophilisation est effectuée à une vitesse de l'ordre de 0,5°C/min, jusqu'à une température inférieure à la température de fusion commençante de la dispersion.

8. Composition susceptible d'être obtenue par le procédé selon l'une des revendications 1 à 7, et se présentant sous forme d'une poudre compacte comprenant une phase grasse et une phase pulvérulente, ladite phase pulvérulente comprenant un premier type de charge, incompactable, et un second type de charge, lesdits premier et second types de charges étant différents, et étant choisis parmi le type lamellaire minéral, le type lamellaire organique, le type sphérique minéral et le type sphérique organique.

9. Composition selon la revendication 8, dans laquelle les charges de type minéral lamellaire sont choisies parmi les talcs ou silicates de magnésium hydratés, les micas ou aluminosilicates, les argiles telles que les séricites, le kaolin ou silicate d'aluminium hydraté, les nitrures de bore, les micatitanes.

10. Composition selon l'une des revendications 8 à 9, dans laquelle les charges de type lamellaire organique compactables sont des poudres de polymères de tétrafluoroéthylène.

11. Composition selon l'une des revendications 8 à 10, dans laquelle la charge de type lamellaire organique incompactable est la lauroyl-lysine.

12. Composition selon l'une des revendications 8 à 11, dans laquelle les charges de type sphérique minéral compactables sont choisies parmi les oxydes de zinc et de titane, le carbonate de calcium précipité, le carbonate et l'hydrocarbonate de magnésium, la silice sphérique non poreuse et l'hydroxyapatite.

13. Composition selon l'une des revendications 8 à 12, dans laquelle les charges de type sphérique minéral incompactables sont choisies parmi les microsphères de silice à porosité ouverte ou creuses, éventuellement imprégnées d'un actif cosmétique, et les microcapsules de verre ou de céramique.

14. Composition selon l'une des revendications 8 à 13, dans laquelle les charges de type organique sphérique compactables sont choisies parmi les savons métalliques dérivés d'acides organiques carboxyliques ayant de 8 à 22 atomes de carbone, tels que le stéarate de zinc, de magnésium ou de lithium, le laurate de zinc, le myristate de magnésium; les poudres de polymères synthétiques non expansés; les poudres de polymères synthétiques, les poudres de résine de silicone; les poudres de matériaux organiques d'origine naturelle.

15. Composition selon l'une des revendications 8 à 14, dans laquelle les charges incompactables de type sphérique organique sont choisies parmi les microsphères microporeuses de polymères, éventuellement imprégnées par des actifs cosmétiques; les microcapsules de polymères éventuellement réticulés.

16. Composition selon l'une des revendications 8 à 15, comprenant après lyophilisation, 2-98% en poids, de préférence 60-97% en poids, de phase pulvérulente.

17. Composition selon l'une des revendications 8 à 16, dans laquelle la phase pulvérulente comprend 1-100% en poids de charges incompactables, de préférence 60-100%, 0-99% en poids de charge compactable, de préférence 0-40%, et 0-50% en poids de pigments, lesdits pourcentages étant donnés par rapport à la phase pulvérulente.

18. Composition selon l'une des revendications 8 à 17, comprenant 2-10% en poids de charges incompactables ayant une densité inférieure à 0,1 g.cm⁻³.

19. Composition selon l'une des revendications 8 à 17, comprenant 2-40% en poids de charges incompactables ayant une densité comprise entre 0,15 et 0,5 g.cm⁻³.

20. Composition selon l'une des revendications 8 à 17, comprenant 30-90% en poids de charges incompactables ayant une densité supérieure à 0,5 g.cm⁻³.

21. Composition selon l'une des revendications 8 à 20, comprenant 0-40% en poids de charges compactables, par rapport au poids total de la composition.

22. Composition selon l'une des revendications 8 à 21, dans laquelle la phase grasse comprend des huiles et/ou des cires d'origine animale, végétale, minérale ou synthétique, seules ou en mélanges.

23. Composition selon l'une des revendications 8 à 22, comprenant 2-98% en poids de phase grasse, de préférence 3-30% en poids, par rapport au poids total de la composition.

24. Composition selon l'une des revendications 8 à 23, comprenant en outre des additifs lipophiles tels que des actifs cosmétiques lipophiles et/ou des ingrédients liposolubles généralement utilisés en cosmétique.

25. Composition selon l'une des revendications 8 à 24, comprenant en outre des additifs hydrosolubles.

26. Composition selon la revendication 25, dans laquelle les additifs hydrosolubles sont choisis parmi
. les adjuvants de formulation tels que les épaississants, notamment les gommes naturelles, par exemple la gomme arabique, la gomme adragante, la gomme de guar; les dérivés de la cellulose, les pectines comme les dérivés de l'acide alginique et du carraghen, les bentonites et les silices colloïdales, les polysaccharides, des macromolécules synthétiques notamment à groupements vinyliques ou acryliques, des matières amylacées, des dérivés phosphorylés d'alcool aliphatique hydroxylé, des triglycérides naturels ou synthétiques estérifiés; les conservateurs tels que le méthylparaben; les agents de modification du pH tels que la triéthanolamine, et/ou
. les actifs cosmétiques tels que les agents antioxydants ou anti-radicaux libres; les agents hydratants ou humectants tels que la glycérine et le collagène; les agents filtres UV tels que la benzophénone.

27. Composition selon l'une des revendications 8 à 26, présentant un toucher très doux et non gras.

28. Composition selon l'une des revendications 8 à 27, présentant une porosité interne supérieure à 2 m²/g.

29. Composition selon l'une des revendications 8 à 28, présentant une porosité interne supérieure à 3 m²/g.

30. Composition selon l'une des revendications 8 à 29, présentant une porosité interne de l'ordre de 4 à 100 m²/g.

## Patentansprüche

1. Verfahren zur Herstellung einer Zusammensetzung, die eine Fettphase und eine Pulverphase enthält, wobei die Pulverphase einen ersten Typ von Füllstoff, der nicht verdichtbar ist, und einen zweiten Typ von Füllstoff enthält, wobei der erste Typ von Füllstoffen und der zweite Typ von Füllstoffen verschieden sind und wobei die Füllstoffe unter den Füllstoffen vom anorganischen lamellaren Typ, vom organischen lamellaren Typ, vom anorganischen kugelförmigen Typ und vom organischen kugelförmigen Typ ausgewählt sind und wobei nach dem Verfahren eine Emulsion der Fettphase in der wässrigen Phase vom Öl-in-Wasser-Typ hergestellt, die Pulverphase in der Emulsion dispergiert, die erhaltene Dispersion in eine Form gegossen und die Dispersion durch Gefriertrocknung getrocknet wird.

2. Verfahren nach Anspruch 1, wobei die nach der Gefriertrocknung erhaltene Dispersion unter Erhalt eines verpressten Pulvers entformt wird.

3. Verfahren nach einem der vorhergehenden Ansprüche, wobei die Emulsion in Gegenwart eines grenzflächenaktiven Stoffes vorzugsweise in einer Menge von 5 bis 15 %, bezogen auf das Gesamtgewicht der Dispersion, hergestellt wird.

4. Verfahren nach einem der vorhergehenden Ansprüche, wobei die Dispersion vor dem Formen 0,1 bis 30 % Fettphase, 0,1 bis 65 % Pulverphase und 30 bis 70 % wässrige Phase enthält.

5. Verfahren nach einem der vorhergehenden Ansprüche, wobei die Dispersion vor dem Formen 1 bis 15 % Fettphase, 1 bis 45 % Pulverphase und 35 bis 55 % wässrige Phase enthält.

6. Verfahren nach einem der vorhergehenden Ansprüche, wobei die Dispersion vor dem Formen eine mit einem Rotationsviskosimeter bei 25 °C gemessene Viskosität von 0,5 bis 30 Pa·s aufweist.

7. Verfahren nach einem der vorhergehenden Ansprüche, wobei die Gefriertrocknung mit einer Geschwindigkeit in der Größenordnung von 0,5 °C/min bis zu einer Temperatur unter der beginnenden Schmelztemperatur der Dispersion durchgeführt wird.

8. Zusammensetzung, die nach dem Verfahren nach einem der Ansprüche 1 bis 7 erhältlich ist und die in Form eines verpressten Pulvers vorliegt, das eine Fettphase und eine Pulverphase enthält, wobei die Pulverphase einen ersten Typ von Füllstoff, der nicht verdichtbar ist, und einen zweiten Typ von Füllstoff enthält, wobei der erste Typ von Füllstoffen und der zweite Typ von Füllstoffen verschieden sind und wobei die Füllstoffe unter den Füllstoffen vom anorganischen lamellaren Typ, vom organischen lamellaren Typ, vom anorganischen kugelförmigen Typ und vom organischen kugelförmigen Typ ausgewählt sind.

9. Zusammensetzung nach Anspruch 8, wobei die Füllstoffe vom Typ der lamellaren anorganischen Füllstoffe unter den Talken oder hydratisierten Magnesiumsilicaten, Glimmern oder Aluminosilicaten, Tonen, wie Sericiten, Kaolin oder hydratisiertem Aluminiumsilicat, Bornitriden und Titanglimmern ausgewählt sind.

10. Zusammensetzung nach einem der Ansprüche 8 bis 9, wobei die Füllstoffe vom Typ der verdichtbaren organischen lamellaren Füllstoffe Pulver von Tetrafluorethylenpolymeren sind.

11. Zusammensetzung nach einem der Ansprüche 8 bis 10, wobei der Füllstoff vom Typ der nicht verdichtbaren organischen lamellaren Füllstoffe das Lauroyllysin ist.

12. Zusammensetzung nach einem der Ansprüche 8 bis 11, wobei die Füllstoffe vom Typ der verdichtbaren anorganischen kugelförmigen Füllstoffe unter Zinkoxid und Titanoxid, gefälltem Calciumcarbonat, Magnesiumcarbonat, Magnesiumhydrogencarbonat, nicht porösem kugelförmigen Siliciumdioxid und Hydroxyapatit ausgewählt sind.

13. Zusammensetzung nach einem der Ansprüche 8 bis 12, wobei die Füllstoffe vom Typ der nicht verdichtbaren anorganischen kugelförmigen füllstoffe unter den offenporigen oder hohlen Mikrokugeln aus Siliciumdioxid, die gegebenenfalls mit einem kosmetischen Wirkstoff imprägniert sind, und Mikrokapseln aus Glas oder Keramik ausgewählt sind.

14. Zusammensetzung nach einem der Ansprüche 8 bis 13, wobei die Füllstoffe vom Typ der verdichtbaren organischen kugelförmigen Füllstoffe unter den Metallseifen, die von organischen Carbonsäuren mit 8 bis 22 Kohlenstoffatomen abgeleitet sind, beispielsweise Zinkstearat, Magnesiumstearat, Lithiumstearat, Zinklaurat und Magnesiummyristat; Pulvern von nicht expandierten synthetischen Polymeren; Pulvern von synthetischen Polymeren; Siliconharzpulvern; und Pulvern organischer Materialien natürlicher Herkunft ausgewählt sind.

15. Zusammensetzung nach einem der Ansprüche 8 bis 14, wobei die nicht verdichtbaren Füllstoffe vom Typ der organischen kugelförmigen Füllstoffe unter den mikroporösen Mikrokugeln von Polymeren, die gegebenenfalls mit kosmetischen Wirkstoffen imprägniert sind; und Mikrokapseln von gegebenenfalls vernetzten Polymeren ausgewählt sind.

16. Zusammensetzung nach einem der Ansprüche 8 bis 15, die nach der Gefriertrocknung 2 bis 98 Gew.-% und vorzugsweise 60 bis 97 Gew.-% Pulverphase enthält.

17. Zusammensetzung nach einem der Ansprüche 8 bis 16, wobei die Pulverphase 1 bis 100 Gew.-% und vorzugsweise 60 bis 100 % nicht verdichtbare Füllstoffe, 0 bis 99 % und vorzugsweise 0 bis 40 % verdichtbaren Füllstoff und 0 bis 50 Gew.-% Pigmente enthält, wobei die Prozentangaben bezogen auf die Pulverphase angegeben sind.

18. Zusammensetzung nach einem der Ansprüche 8 bis 17, die 2 bis 10 Gew.-% nicht verdichtbare Füllstoffe mit einer Dichte unter 0,1 g·cm⁻³ enthält.

19. Zusammensetzung nach einem der Ansprüche 8 bis 17, die 2 bis 40 Gew.-% nicht verdichtbare Füllstoffe mit einer Dichte von 0,15 bis 0,5 g·cm⁻³ enthält.

20. Zusammensetzung nach einem der Ansprüche 8 bis 17, die 30 bis 90 Gew.-% nicht verdichtbare Füllstoffe mit einer Dichte über 0,5 g·cm⁻³ enthält.

21. Zusammensetzung nach einem der Ansprüche 8 bis 20, die 0 bis 40 Gew.-% verdichtbare Füllstoffe, bezogen auf das Gesamtgewicht der Zusammensetzung, enthält.

22. Zusammensetzung nach einem der Ansprüche 8 bis 21, wobei die Fettphase Öle und/oder Wachse tierischer, pflanzlicher, mineralischer oder synthetischer Herkunft einzeln oder im Gemisch enthält.

23. Zusammensetzung nach einem der Ansprüche 8 bis 22, die 2 bis 98 Gew.-% und vorzugsweise 3 bis 30 Gew.-% Fettphase, bezogen auf das Gesamtgewicht der Zusammensetzung, enthält.

24. Zusammensetzung nach einem der Ansprüche 8 bis 23, die ferner fettlösliche Zusatzstoffe, wie lipophile kosmetische Wirkstoffe, und/oder fettlösliche Bestandteile, die gewöhnlich in der Kosmetik verwendet werden, enthält.

25. Zusammensetzung nach einem der Ansprüche 8 bis 24, die ferner wasserlösliche Wirkstoffe enthält.

26. Zusammensetzung nach Anspruch 25, wobei die wasserlöslichen Zusatzstoffe ausgewählt sind unter:
· Hilfsstoffen für die Formulierung, beispielsweise Verdickungsmitteln, insbesondere natürlichen Gummen, beispielsweise Gummi arabicum, Tragant und Guargummi; Cellulosederivaten, Pektinen, wie beispielsweise Derivaten von Alginsäure und Carrageen, Bentoniten und kolloidalen Kieselsäuren, Polysacchariden, synthetischen Makromolekülen insbesondere mit Vinyl- oder Acrylgruppen, Stärkeerzeugnissen, phosphorylierten Derivaten von hydroxylierten aliphatischen Alkoholen, natürlichen oder synthetischen veresterten Triglyceriden; Konservierungsmitteln wie Methylparaben; und Stoffen zur Veränderung des pH-Werts, wie Triethanolamin, und/oder
kosmetischen Wirkstoffen, wie Antioxidantien oder Radikalfängern für freie Radikale; Hydratisierungsmitteln oder
Feuchthaltemitteln, wie Glycerin und Kollagen; und UV-Filtern wie Benzophenon.

27. Zusammensetzung nach einem der Ansprüche 8 bis 26, die sich weich und nicht fettig anfühlt.

28. Zusammensetzung nach einem der Ansprüche 8 bis 27, die eine innere Porosität über 2 m²/g aufweist.

29. Zusammensetzung nach einem der Ansprüche 8 bis 28, die eine innere Porosität über 3 m²/g aufweist.

30. Zusammensetzung nach einem der Ansprüche 8 bis 29, die eine innere Porosität in der Größenordnung von 4 bis 100 m²/g aufweist.

## Claims

1. Process for preparing a composition comprising a fatty phase and a pulverulent phase, the said pulverulent phase comprising a first type of filler, which is noncompactable, and a second type of filler, the said first and second types of filler being different, and being chosen from the inorganic lamellar type, the organic lamellar type, the inorganic spherical type and the organic spherical type, in which process an oil-in-water type emulsion of the fatty phase in an aqueous phase is prepared, the pulverulent phase is dispersed in the said emulsion, the dispersion obtained is poured into a mould and the said dispersion is dried by freeze-drying.

2. Process according to Claim 1, in which the dispersion obtained after freeze-drying is unmoulded so as to obtain a compact powder.

3. Process according to one of the preceding claims, in which the emulsion is prepared in the presence of a surfactant, preferably in a quantity of 5-15% relative to the total weight of the dispersion.

4. Process according to one of the preceding claims, in which the dispersion before moulding comprises 0.1-30% of fatty phase, 0.1-65% of pulverulent phase and 30-70% of aqueous phase.

5. Process according to one of the preceding claims, in which the dispersion before moulding comprises 1-15% of fatty phase, 1-45% of pulverulent phase and 35-55% of aqueous phase.

6. Process according to one of the preceding claims, in which the dispersion has, before moulding, a viscosity of 0.5-30 Pa.s, measured at 25°C with the aid of a rotary viscometer.

7. Process according to one of the preceding claims, in which the freeze-drying is carried out at a rate of the order of 0.5°C/min up to a temperature of less than the onset of melting temperature of the dispersion.

8. Composition which can be obtained by the process according to one of Claims 1 to 7, and provided in the form of a compact powder comprising a fatty phase and a pulverulent phase, the said pulverulent phase comprising a first type of filler, which is noncompactable, and a second type of filler, the said first and second types of filler being different, and being chosen from the inorganic lamellar type, the organic lamellar type, the inorganic spherical type and the organic spherical type.

9. Composition according to Claim 8, in which the fillers of the lamellar inorganic type are chosen from talcs or magnesium silicate hydrates, micas or aluminosilicates, clays such as sericites, kaolin or aluminium silicate hydrate, boron nitrides, and mica-titaniums.

10. Composition according to one of Claims 8 to 9, in which the fillers of the organic lamellar type which are compactable are powders of tetrafluoroethylene polymers.

11. Composition according to one of Claims 8 to 10, in which the filler of the organic lamellar type which is noncompactable is lauroyllysine.

12. Composition according to one of Claims 8 to 11, in which the fillers of the inorganic spherical type which are compactable are chosen from zinc and titanium oxides, precipitated calcium carbonate,. magnesium carbonate and hydrocarbonate, nonporous spherical silica and hydroxyapatite.

13. Composition according to one of Claims 8 to 12, in which the fillers of the inorganic spherical type which are noncompactable are chosen from silica microspheres having an open or hollow porosity, optionally impregnated with a cosmetic active agent, and glass or ceramic microcapsules.

14. Composition according to one of Claims 8 to 13, in which the fillers of the spherical organic type which are compactable are chosen from metallic soaps derived from organic carboxylic acids having from 8 to 22 carbon atoms, such as zinc, magnesium or lithium stearate, zinc laurate, magnesium myristate; powders of nonexpanded synthetic polymers; powders of synthetic polymers, powders of silicone resin; powders of organic materials of natural origin.

15. Composition according to one of Claims 8 to 14, in which the noncompactable fillers of the organic spherical type are chosen from microporous microspheres of polymers, optionally impregnated with cosmetic active agents; microcapsules of optionally crosslinked polymers.

16. Composition according to one of Claims 8 to 15, comprising, after freeze-drying, 2-98% by weight, preferably 60-97% by weight, of pulverulent phase.

17. Composition according to one of Claims 8 to 16, in which the pulverulent phase comprises 1-100% by weight of noncompactable fillers, preferably 60-100%, 0-99% by weight of compactable filler, preferably 0-40%, and 0-50% by weight of pigments, the said percentages being given relative to the pulverulent phase.

18. Composition according to one of Claims 8 to 17, comprising 2-10% by weight of noncompactable fillers having a density of less than 0.1 g.cm⁻³.

19. Composition according to one of Claims 8 to 17, comprising 2-40% by weight of noncompactable fillers having a density of between 0.15 and 0.5 g.cm⁻³.

20. Composition according to one of Claims 8 to 17, comprising 30-90% by weight of noncompactable fillers having a density greater than 0.5 g.cm⁻³.

21. Composition according to one of Claims 8 to 20, comprising 0-40% by weight of compactable fillers, relative to the total weight of the composition.

22. Composition according to one of Claims 8 to 21, in which the fatty phase comprises oils and/or waxes of animal, plant, mineral or synthetic origin, alone or as mixtures.

23. Composition according to one of Claims 8 to 22, comprising 2-98% by weight of fatty phase, preferably 3-30% by weight, relative to the total weight of the composition.

24. Composition according to one of Claims 8 to 23, comprising, in addition, lipophilic additives such as lipophilic cosmetic active agents and/or fat-soluble ingredients generally used in cosmetics.

25. Composition according to one of Claims 8 to 24, comprising, in addition, water-soluble additives.

26. Composition according to Claim 25, in which the water-soluble additives are chosen from
. formulation adjuvants such as thickeners, in particular natural gums, for example gum arabic, gum tragacanth, guar gum, cellulose derivatives, pectins such as derivatives of alginic acid and of carrageenan, bentonites and colloidal silicas, polysaccharides, synthetic macromolecules, especially containing vinyl or acrylic groups, starchy materials, phosphorylated derivatives of a hydroxylated aliphatic alcohol, esterified natural or synthetic triglycerides; preservatives such as methylparaben; pH-modifying agents such as triethanolamine, and/or
. cosmetic active agents such as antioxidants or anti-free-radical agents; moisturizing agents or humectants such as glycerine and collagen; UV-screening agents such as benzophenone.

27. Composition according to one of Claims 8 to 26, having a very smooth and non fatty feel.

28. Composition according to one of Claims 8 to 27, having an internal porosity greater than 2 m²/g.

29. Composition according to one of Claims 8 to 28, having an internal porosity greater than 3 m²/g.

30. Composition according to one of Claims 8 to 29, having an internal porosity of the order of 4 to 100 m²/g.
